(19) **Europäisches Patentamt**
**European Patent Office**
**Office européen des brevets**

(11) **EP 2 084 974 A1**

(12) **EUROPEAN PATENT APPLICATION**

(43) Date of publication:
**05.08.2009 Bulletin 2009/32**

(21) Application number: **08101169.4**

(22) Date of filing: **31.01.2008**

(51) Int Cl.:
*A23L 1/30* (2006.01)          *A23L 1/308* (2006.01)
*A23L 1/09* (2006.01)          *A23L 2/52* (2006.01)
*A23L 1/054* (2006.01)          *C12N 9/10* (2006.01)
*C12N 9/24* (2006.01)          *C12P 19/04* (2006.01)
*C12P 19/18* (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Bayer CropScience AG**
**40789 Monheim (DE)**

(72) Inventors:
• **Pilling, Jens**
  **44287 Dortmund (DE)**
• **Frohberg, Claus**
  **14532 Kleinmachnow (DE)**

(74) Representative: **Beyer, Andreas**
  **Hermannswerder 20a**
  **14473 Potsdam (DE)**

(54) **The use of alternan-oligosaccharide as a degradation-resistant ingredient for acidic beverages**

(57)  The present invention is directed to the use of an alternan-oligosaccharide as a degradation-resistant ingredient for acidic beverages and to a beverage comprising alternan-oligosaccharide as a degradation-resistant ingredient.

EP 2 084 974 A1

**Description**

The present invention is directed to the use of an alternan-oligosaccharide as a degradation-resistant ingredient for acidic beverages and to a beverage comprising alternan-oligosaccharide as a degradation-resistant ingredient.

Alternan is a polysaccharide composed of glucose units. The glucose units are linked to each other via $\alpha$-1,3- and $\alpha$-1,6-glycosidic bonds, and said two types of bonds predominantly appear alternatingly.

Alternan-oligosaccharides have been described as prebiotic ingredients. US patent 7,182,954 discloses that oligosaccharides produced by an alternansucrase enzyme catalyzed reaction of sucrose with various acceptor sugars are effective as prebiotics for controlling enteric bacterial pathogens. Populations of enteropathogenic bacteria may be substantially reduced or inhibited by treatment of an animal with a composition comprising one or more of these oligosaccharides in an amount effective to promote the growth of beneficial bacteria (e.g. Lactobacilli, Bifidobacteria).

The publication WO2006088884 provides with methods of making substantially clear low-glycemic syrups (LGS) that comprise alternan oligosaccharides. These syrups have a relatively low glycemic index and are additionally useful in applications where increased clarity is desired. These qualities are particularly beneficial in food and beverage formulations.

However, the need persists for prebiotic food ingredients which retain their beneficial properties in low pH environments as it is the case in acidic beverages.

Therefore, it is an object of the present invention to provide with a pH stable prebiotic and/or low glycemic and/or soluble fiber ingredient for beverages.

A further object of the present invention is to provide with a pH and temperature-stable prebiotic and/or low glycemic and/or soluble fiber ingredient for beverages since some beverage processes call for hot filling.

The present invention is directed to the use of an alternan-oligosaccharide as a degradation-resistant ingredient for acidic beverages. Alternan-oligosaccharides are deemed to have prebiotic properties. In the present invention it is shown that alternan-oligosaccharides are not degraded in acidic environment and could therefore retain their prebiotic properties when added to beverages with a low pH. In similar manner, the low glycemic properties and soluble fiber properties of alternan oligosaccharides are retained in acidic beverages, since no glucose is released from the alternan-oligosaccharide molecules.

Alternan according to the present invention is a polysaccharide composed of glucose units. The glucose units are linked to each other via $\alpha$-1,3- and $\alpha$-1,6-glycosidic bonds, and said two types of bonds predominantly appear alternatingly. Alternan-oligosaccharides are wellknown from the state of the art and for example disclosed together with manufacturing processes in WO 00/47727, US 7,182,954 WO 2006/088884, and Cote and Robyt 1982, Carbohydrate Research, 111:127-142 which are incorporated by reference in their entirety in the present description.

In the present invention the singular-term "alternan-oligosaccharide" designates both monodisperse alternan-oligosaccharides with molecules of only one degree of polymerization (DP) as well as polydisperse alternan-oligosaccharides with molecules having different degrees of polymerization.

Alternan-oligosaccharide in the definition of in the present invention has a degree of polymerization in the range of 3 - 20. In a preferred embodiment, alternan-oligosaccharide according to the invention has a degree of polymerization (DP) in the range of 3 - 15, more preferably in the range of 3 - 12, still more preferably in the range of 3 - 10, and most preferably in the range of 3 - 8.

Alternan polymer can be produced as disclosed in WO 00/47727. Alternan-oligosaccharides can be produced from alternan polymer by degradation of alternan polymer under appropriate conditions. The degradation can for example be an enzymatic degradation of alternan polymer or degradation under acidic conditions, preferably with heating.

Alternan-oligosaccharides can also be produced by the reaction of sucrose with an acceptor molecule in presence of alternansucrase enzyme, which is preferred according to the present invention.

Alternan-oligosaccharides may be prepared by a method, wherein

a) a sucrose containing solution is contacted with a catalytically effective amount of alternansucrase enzyme and acceptor molecules under conditions permitting the conversion of sucrose to alternan-oligosaccharide and fructose; and

b) alternan-oligosaccharide and fructose are isolated from the solution.

The reaction may be conducted between room temperature and 37°C and at a pH between about 5 and 7, and may be allowed to proceed until the sucrose has been essentially consumed. Detailed reaction conditions are disclosed in WO 00/47727, US 7,182,954 and in the appended example. The product is usually obtained as a syrup which may further be purified, i.e. by membrane filtration, and/or dried.

The acceptor molecule is understood to mean a molecule at which an alternansucrase is able to catalyze a chain-extending reaction. The acceptor which can be added to the reaction mixture at the beginning of the reaction is preferably a carbohydrate or a carbohydrate derivative. The use of external acceptors leads to the production of low molecular alternan-oligosaccharides. The carbohydrate acceptor is preferably a saccharide selected from the group consisting of maltose, isomaltose, maltitol, (iso)maltotriose and methyl-$\alpha$-D-glucan. Other preferred acceptor molecules are glucose, gentiobiose, raffinose, melibiose, isomaltitol, isomaltooligosaccharide, theanderose, kojibiose, glucosyl trehaloses, cellobiose, maltotetraose, nigerose, lactose, panose or mixtures thereof.

Depending upon the particular acceptor selected, the glucosyl units will generally be added through an $\alpha$(1,6) linkage, or through an $\alpha$(1,3) linkage if an $\alpha$(1,6) linkage is already present. Alternan-oligosaccharides are obtained which have a lower molecular weight than the alternan that can be prepared in the absence of external acceptors. The reaction will typically produce a mixture of oligosaccharides having different degrees of polymerization (DP). If alternan-oligosaccharides are produced by the reaction of sucrose with an acceptor molecule, the degree of polymerization (DP) is defined as the number of D-glucosyl units added onto the original acceptor molecule plus the number of monosaccharide units in the original acceptor oligosaccharide.

The extent of the degree of polymerization may vary with the concentrations and the relative ratio of sucrose and acceptor oligosaccharide. The reaction product will generally be composed of a mixture of oligosaccharides having different degrees of polymerization. At a relatively high sucrose:acceptor ratio, more glucosyl units are transferred into glucan and products with higher degree of polymerization are obtained (i.e. the relative amounts of the high DP oligosaccharides in the product will be increased). In contrast, at a low sucrose:acceptor ratio, the predominant reaction product is that resulting from the transfer of a single glucosyl unit to the acceptor. Thus, the yields of oligosaccharides of a desired degree of polymerization may be optimized by varying the sucrose:acceptor ratio. The precise sucrose:acceptor ratios for a desired degree of polymerization will vary with the particular acceptor oligosaccharide and may be readily determined by routine experimentation.

Alternansucrase for use herein may be obtained from a variety of microorganisms, preferably strains of Leuconostoc and particularly strains of L. mesenteroides, as for example disclosed in WO 00/47727. In a preferred embodiment, the enzyme is produced by strains of which secrete a high proportion of alternansucrase to dextransucrase such as described by Leathers et al. (U.S. Pat. No. 5,702,942, the contents of which are incorporated by reference herein). In another preferred embodiment the alternansucrase enzymes that can be used to produce alternan-oligosaccharides include Leuconostoc mesenteroides strains NRRL B 1355, 23185, 23186, 23188, 23311, 21297, 30821, 30894 These enzymes can be additionally cloned and expressed recombinantly, such as described in Gilles Joucla, Doctroal Dissertation, Ingenier INSA, Toulouse, France, 2003.

Production of the alternansucrase may be conducted by culture of any of the above-mentioned microorganisms using conventional techniques and under aerobic conditions which are effective to promote growth and production of the enzyme such as described in Leathers et al. or the example herein below. Following culture, the enzyme may be isolated or separated from the microorganisms using conventional techniques, such as by centrifugation or filtration.

The term "degradation-resistant" means that the degree of polymerization of alternan-oligosaccharide does not measurably (High performance anion-exchange chromatography - HPAEC or Gel permeation chromatography - GPC-RI) decrease in acidic solution of pH 6 or below, preferably pH 3 or below, over a time period of at least 8 weeks when the solution is stored at a temperature of up to 32°C. A main product of degradation of alternan-oligosaccharides is glucose and the degradation mechanism normally takes place by hydrolysis of oligoalternan chains. Because of its degradation-resistance, alternan-oligosaccharide is excellently suitable as a degradation-resistant prebiotic and/or low glycemic and/or soluble fiber ingredient for acidic beverages. The acidic beverage is defined as a beverage having a pH below 7. The acidic beverage has preferably a pH of 6 or below, more preferably a pH of 3.5 or below. In still another preferred embodiments the acidic beverage has a pH in the range of 1 - 6, more preferably 1 - 5, still more preferably 1 - 4 and most preferably 1.5 - 3.5.

In the present invention it can also be shown that alternan-oligosaccharide is not measurably degraded in aqueous environment of pH 3 or above when heated to a temperature of up to 120°C for 1 hour. In aqueous environment of pH 1.5 alternan-oligosaccharide is not measurably degraded when heated to a temperature of up to 95°C for 1 hour. No measurable degradation in this context means that no increase of glucose is detected (High performance anion-exchange chromatography - HPAEC or Gel permeation chromatography - GPC-RI) in a heated sample as degradation product of alternan-oligomers.

This property of alternan-oligosaccharide is beneficial in the manufacturing process of a beverage since the beverages are sometimes heated to higher temperatures during the process. Some beverage processes call for hot filling. This involves heating the beverage to 80-90°C, holding for about 10 minutes at that temperature, cooling to about 65°C and then bottling. The alternan-oligosaccharide can withstand this heat abuse for at least about 10 minutes without visible effects.

The invention is further directed to beverages comprising the above-described alternan-oligosaccharide as a degradation-resistant prebiotic and/or low glycemic and/or soluble fiber ingredient. The beverage is preferably an acidic beverage with a pH in the ranges as defined above. The beverage is preferably selected from fruit juices, energy drinks, lemonades, sherbets, sodas, soft drinks, and flavored waters.

Alternan-oligosaccharide is preferably added to the beverage according to the invention in an amount of 1-20 weight-% based on the total weight of the beverage formulation, more preferably in an amount of 1-10 weight-%, still more preferably in an amount of 1-5 weight-%.

The beverage according to the invention is preferably a clear beverage. Besides its degradation resistance, a further beneficial property of alternan-oligosaccharide is its ability to retain the clarity of a clear beverage when it is added to a clear beverage formulation as a prebiotic and/or low glycemic and/or soluble fiber ingredient. Clarity can be determined using the test procedure described in WO2006/088884 or evaluated visually on a qualitative basis, as shown in the appended examples.

In another aspect, the invention provides with methods for manufacturing a beverage as described above. In one method for manufacturing a beverage, alternan-oligosaccharide is blended with other ingredients to form a premix which is subsequently added to the water basis of the beverage. Alternan-oligosaccharide can be blended with one or more further ingredients such as vitamins, minerals, sugar alcohols, high intensity sweeteners, flavors, flavor enhancers, acids, as citric acid or malic acid, and conventional sweeteners. In another embodiment only alternan-oligosaccharide added to the ready made beverage. In principle, all manufacturing methods for beverages which are known to an expert skilled in the art can be employed without limitation.

The following examples are intended only to further illustrate the invention and are not intended to limit the scope of the invention which is defined by the claims.

EXAMPLES

Literature:

- Arguello-Morales MA, Remaud-Simeon M, Pizzut S, Sarcabal P, Willemot R and Monsan P (2000) Sequence analysis of the gene encoding alternansucrase, a sucrose glucosyltransferase from Leuconostoc mesenteroides NRRL B-1355. FEMS Microbiol 1 Lett 182: 81-85.
- Degenkolb J, Takahashi M, Ellestad GA, Hillen W, 1991:Antimicrob. Agents Chemother. 35, No 8, 1591-1595 Structural requirements of tetracycline-Tet repressor interaction: Determination of equilibrium binding constants for tetracycline analogues with the Tet repressor.
- Dotto GP, Horiuchi K, Zinder ND (1982) Initiation and termination of phage fl plus-strand synthesis. Proc Natl Acad Sci U S A. 79:7122-7126.
- Horn, U., Strittmatter, W., Krebber, A., Knüpfer, U., Kujau, M., Wenderoth, R., Müller, K., Matzku, S., Plückthun, A., and Riesenberg, D. (1996) High volumetric yields of functional dimeric miniantibodies in Escherichia coli, using an optimized expression vector and high-cell-density fermentation under non-limited growth conditins. Appl. Microbio. Biotechnol. 46, 524-532.
- Jeong SH, Bae IK, Lee JH, Sohn SG, Kang GH, Jeon GJ, Kim YH, Jeong BC and Lee SH. (2004) Molecular Characterization of Extended-Spectrum Beta-Lactamases Produced by Clinical Isolates of Klebsiella pneumoniae and Escherichia coli from a Korean Nationwide Survey. J Clin Microbiol 42: 2902-2906.
- López-Muguía, A., Pelenc, V., Remaud, M., Biton, J.1, Michel, J.M., Lang, C., Paul, F., Monsan, P. (1993). Production and purification of alternansucrase, a glucosyltransferase from Leuconostoc mesenteroides NRRL B-1355, for the synthesis of oligoalternan: Enzyme Microb. Technol., 15, 77-85.
- Schmidt TGM and Skerra A (1993). The random peptide library-assisted engineering of a C-terminal affinity peptide, useful for the detection and purification of a functional Ig Fv fragment. Protein Eng 6: 109-122.
- Skerra, A (1994). Use of the tetracycline promoter for the tightly regulated production of a murine antibody fragment in Escherichia coli. Gene 151, 131-135.

1. Fermentative production of alternansucrase from Leuconostoc mesenteroides

For heterologous expression of the alternansucrase (ARS) from Leuconostoc mesenteroides strain NRRL B-1355, the gene encoding alternansucrase has been isolated, fused to a Strep-tag (IBA BioTAGnology, Göttingen, Germany) and cloned into vector pAI-B-AlSu under the control of a tetrayclince-inducible promoter.

The genetically modified E. coli strain DH5a pAIB-AlSu Q29 employed for fermentation harbours plasmid pAI-B-AlSu for cytoplasmic expression of alternansucrase from Leuconostoc mesenteroides. Vector pAI-B-AlSu is essentially derived from plasmid pASK-IBA-3 (purchased from IBA Göttingen; www.iba-go.com). It contains the coding sequence of alternansucrase derived from Leuconostoc mesenteroides strain NRRL B-1355 fused to a 8 aminoacid peptide strep-tag at the C-terminal end. The strep-tag is linked to the protein through a 2 aminoacid linker. Expression of alternansucrase is under the transcriptional control of the tetA promoter/operator and repressor. The tetA promoter is tightly regulated by the tet repressor which is encoded on the same plasmid and is constitutively expressed from the β-lactamase promoter.

In this way, expression of alternansucrase is stringently repressed until efficient chemical induction by tetracycline or anhydrotetracycline, AHT, (Degenkolb et al.; 1991).

Vector pAl-B-AlSu contains the following genetic elements:

| Nt Positions | Orientation | Origin |
|---|---|---|
| 37-72 | Clockwise | PTet: Tet Promoter from transposon Tun10 (Skerra, 1994) |
| 139-6309 | Clockwise | asrLm: Coding sequence of the Altemansucrase gene from *Leuconostoc mesenteroides* NRRL B-1355. (Arguello-Morales *et al.,* 2000) |
| 6310-6315 | Clockwise | SA linker encoding 2 Amino Acids: Serine and Alanine |
| 6316-6345 | Clockwise | StrepTag: sequence encoding a 8 AA peptide selected from a genetic fusion peptide library for its ability to bind to streptavidin (Schmidt and Skerra, 1993) |
| 6439-6877 | Clockwise | f1 origin: origin of replication from filamentous phage f1 (Dotto *et al.,* 1982) |
| 7026-7886 | Clockwise | AmpR: Coding sequence of β-lactamase from Escherichia coli, conferring Ampicillin resistance (Jeong *et al.,* 2004) |
| 7896-8519 | Clockwise | TetR: Tet repressor domain from Transposon Tn10 (Skerra, 1994) |

For fermentation, vector pAl-B-AlSu is transformed in E. coli K12 DH5α and bacterial cells harbouring the vector are selectively grown for 12 h at 37°C to an OD600 of 65 in mineral medium (Horn et al., 1996) supplemented with ampicillin (100 μg/ml). Expression of the alternansucrase is induced by the addition of anhydrotetracyclin (0.2 mg/l) and further cultivation for 5 h at 25°C to an OD600 of 140. For purification of the enzyme, the bacterial cells are harvested by centrifugation (20000 rpm; 20 min) and solubilized in resuspension buffer (100 mM NaAc, pH 5.3).

The cells are disrupted using a high pressure homogenizer (two cycles, 1200 bar). Bacterial nucleic acid is degraded by DNase/RNase (3 mg/1) treatment and the resulting extract is centrifuged (3,800 g for 15 min at 4°C) to harvest the insoluble cell matter including the bacterial inclusion bodies.

The supernatant is discarded and the pellet is resuspended 8 M urea, 50 mM NaAc buffer, pH 5.3 and kept on ice while shaking for one hour. Subsequently, the remaining debris is removed by centrifugation at 10.000 g for 15 min. Renaturation is then performed by 20-fold dilution in 0.5 M urea, 2.5 mM CaCl2, 100 mM NaAc, pH 5.3. Aliquots of the mixture are immediately frozen in liquid nitrogen and stored at -20 °C.

Activity of alternansucrase can be determined as described by Lopez-Munguia et al., 1993.

2. Production of alternan-oligosaccharides with acceptor molecule maltose (maltose oligosaccharides)

For the production of maltose oligosaccharides, the purified alternansucrase enzyme as described in 1 is incubated with 10 % sucrose (w/v) and 4.5-5% maltose (w/v) in a 50 mM sodium acetate buffer pH 5.3 at room temperature for approximately 72 hours. For the incubation, 50 units of alternansucrase per litre reaction mix are used. Alternan, which accumulates as a by-product, is precipitated by adding analytical grade ethanol to a final concentration of 50 % (v/v). The mixture is centrifuged at 4000 rpm for 10 min and the resulting precipitate is discarded.

Maltose oligosaccharide is prepared by further concentrating the obtained precipitate using a vacuum vaporizer (Buechi Rotavapor R-220) to about 70° brix. Maltose oligosaccharide is obtained as a syrup which is further purified and dried to obtain the final product.

3. Application testing in flavored waters

Two retail flavored waters were testes in an application study. Alternan-oligosaccharide (also designated hereinafter as "maltose oligosaccharide" because auf maltose acceptor) produced as described in Example 2 was used for the tests.

| Beverage Name | pH | Comments |
|---|---|---|
| Metro Mint® (Soma Beverage Co.) | 5.87 | Clear, colorless |
| Fruit20® (Kraft) | 3.02 | Clear, colorless |

Set Up

- Storage Temperatures: -17.8°C, 4.4°C, 21°C, 32.2°C
- Evaluation Points: 0 Time, 1 Week, 4 Weeks, 8 Weeks

Beverage Production

- Alternan-oligosaccharide was combined with the flavored waters in a large batch.
- Alternan-oligosaccharide was added to the water while agitating with a Lightning Mixer at 700 rpm. After the entire amount of alternan-oligosaccharide was added, the beverage was mixed for an additional 1.5 minutes.
- The percentages used in the preliminary screening were used for the shelf life. Each variable is present at a level to give a 5 gram per serving level of fiber.
- After mixing, the beverage was aliquoted to separate bottles for storage. The bottles were placed in storage at each temperature.
- One bottle was used for each evaluation point.
- The samples were allowed to come to room temperature before evaluation.

The following protocols were used to evaluate the beverages at each interval in the shelf life study.

3.1 Specific gravity - BRIX

- Bausch & Lomb Abbe-3L Refractometer

- Samples were always at room temperature so temperature control was not used.

The brix of the beverages containing maltose-oligosaccharide did not change during the shelf life period of 8 weeks at different storage temperatures (-17.8°C, 4.4°C, 21°C, 32.2°C). Even when precipitation was noted, the brix was not significantly affected. Beverages were always shaken before measurement.

3.2 Color

- 200 ml sample size in 250 ml Pyrex beaker.
- White filter paper was placed over the top of the beaker
- Readings were taken through the bottom of the beaker making sure that the glass was clean.
- Color was evaluated visually and comments made.

The maltose-oligosaccharide did not develop significant color over the shelf life. The maltose-oligosaccharide sample in 32,2°C storage did show a very light yellow color at 8 weeks in the acidic beverage.

3.3 Turbidity and formation of precipitate

Turbidity and precipitation was evaluated visually in the samples over the shelf life, with samples stored at different temperatures.
Maltose-oligosaccharide did cause a slight cloudiness in both water systems. The turbidity did not appear to be significantly affected by time and or temperature.

|  | Average Cloudiness Score | Comments |
|---|---|---|
| maltoseoligosaccharide | 0.5 to 1.0 | very slight. |
| Scale: 0 = clear, 1= slight, 2 = slight + , 3 = moderate, 4 = moderate +, 7 = very heavy | | |

The maltose-oligosaccharide did not form a precipitate under any of the storage conditions.

3.4 Hot Fill Simulation - Abuse Condition

Some beverage processes call for hot filling. This involves heating the beverage to 82°C, holding for 10 minutes at that temperature, cooling to 65°C and then bottling. The maltose-oligosaccharide beverages were exposed to this heat abuse.

Process

- 500 ml of a 2.60% solution of maltose-oligosaccharide in Metro Mint or Fruit$_2$O water was placed in the top of a double boiler.
- Heated to between 82°C and 87.8°C
- Held for 10 minutes at that temperature
- Cooled liquid to 65.6°C
- Bottled
- Beverages observed for 72 hours. Measured for viscosity, pH, and Brix.

Results:

|  | Viscosity (cp) | pH | Brix | Comments |
|---|---|---|---|---|
| Fruit$_2$O Control | 2.16 | 2.89 | 2.8 | |
| Fruit$_2$O Hot fill | 2.16 | 2.94 | 3.0 | No obvious change in appearance. |
| Metro Mint Control | 2.13 | 3.95 | 2.9 | |
| Metro Mint Hot Fill | 2.19 | 4.17 | 2.9 | No obvious change in appearance. |

The maltose-oligosaccharide was tolerant to the hot fill conditions tested.

3.5 Resistance to degradation

For the measurements all samples were diluted (total starting volume 1 ml) und neutralized if necessary (Fruit2O: + 100$\mu$l 150mM NaOH).
High performance anion-exchange chromatography - HPAEC: 1:100; injection volume: 25$\mu$l; HPAEC-PAD: glucan program 45min
Gel permeation chromatography - GPC: Metromint: 1:5 with DMSO, Fruit2O samples (after neutralisation) 1:2.5

Results:

In the GPC profile no change was detected over the shelf life of the sample (8 weeks).
HPAEC: no changes were detected in fractions over DP3. In Metromint a slight increase in glucose over shelf life was detected at 32.2°C. In Fruit2O sucrose decreased and glucose and fructose increased during shelf life at 32.2°C

| Metromint | | | Fruit2O | | |
|---|---|---|---|---|---|
| storage temperature/time | MW g/mol | DP | storage temperature/time | MW | DP |
| -17.8°C / 0 weeks | 920 | 6 | -17.8°C / 0 weeks | 896 | 6 |
| -17.8°C / 1 week | 916 | 6 | | | |
| -17.8°C / 4 weeks | 910 | 6 | -17.8°C / 4 weeks | 895 | 6 |
| | | | -17.8°C / 8 weeks | 898 | 6 |
| | | | | | |
| 32.2°C / 0 weeks | 909 | 6 | 32.2°C / 0 weeks | 901 | 6 |
| 32.2°C / 1 week | 906 | 6 | 32.2°C / 1 week | 900 | 6 |
| 32.2°C / 4 weeks | 909 | 6 | 32.2°C / 4 weeks | 903 | 6 |
| 32.2°C / 8 weeks | 910 | 6 | 32.2°C / 8 weeks | 938 | 6 |
| In DP calculations 162 g/mol was used as MW of monomer | | | | | |

4. <u>Resistance to degradation in carbonated beverages</u>

Three retail beverages (Coca Cola®, Fanta® and Orange lemonade) were prepared with alternanoligosaccharide (also designated as maltose-oligosaccharide because of maltose acceptor molecule) and competitive products for the shelf life screening.

Maltose-oligosaccharide was produced according to a procedure similar to Ex. 2, except that 30% sucrose and 15% maltose were used and the ethanol precipitation step was omitted. The product was purified by filtration techniques and maltose-oligosaccharide was obtained as a syrup containing about 15 weight-% fructose.

Set Up

- Storage Temperatures: 4°C, RT (24°C), 37°C
- Evaluation Points: 0 time, 4 days, 1 week, 2 weeks, 4 weeks, 8 weeks

Beverage Production

- Maltose-oligosaccharide was combined with the beverage in 50g batch.
- 2.5% of maltose-oligosaccharide was used in the screening. Maltose-oligosaccharide was added to the beverage in a 50ml Falcon. After the entire amount of fiber 5g of water was added and the beverage was mixed.
- After mixing, the 50g batch was distributed to small beverage amounts (ca. 3g) and put in small bottles. Those small bottles were used for the shelf life storage at the different conditions.
- One bottle was used for each evaluation point.
- After each storage time the samples were visually evaluated and then frozen at -18°C for GPC-RI and HPAEC-PAD measurement.

GPC-RI and HPAEC-PAD results:

The beverages have been measured for degradation of maltose-oligosaccharide using GPC-RI and HPAEC-PAD at BBS. In none of the three beverages maltose-oligosaccharide showed any degradation during storage over the investigated time range at the different temperatures. The only alteration was to be seen in sucrose content. This effect was most drastic in beverages with lowest pH at 37°C (Coca Cola® and Fanta®). Sucrose content of orange lemonade also decreased, but to a lower amount.

5. <u>Heat and acid stability of alternanoligosaccharide</u>

Material: Maltose-oligosaccharide was produced according to a procedure similar to Ex. 2, except that 30% sucrose and 15% maltose were used and the ethanol precipitation step was omitted. The product was purified by filtration techniques and maltose-oligosaccharide was obtained as a syrup containing about 15 weight-% fructose, 68.8° BRIX (internal reference M2).

The alternan-oligosaccharide contained about 15 weight-% of fructose. For preparation of samples all amounts were calculated in such manner that > 5% pure maltose-oligosaccharide was contained in the final sample.

Samples:

- alternan-oligosaccharide in 0.1M HAc - pH 3
- alternan-oligosaccharide in 0.02M HCl - pH 1.5

The samples were incubated for 10 minutes at temperatures of 20, 40, 60, 80, and 95°C, respectively, 1 hour at 120°C, and then cooled down for 10 min. 100 µl volumes were taken from each sample and neutralized:

$$0.1M \text{ HAc: } 100\mu l + 100\mu l \text{ N-Mix HAc } (450\mu l \text{ 1M NaOH ad 10ml})$$

$$0.02M \text{ HCl: } 100\mu l + 100\mu l \text{ N-Mix HCl } (175\mu l \text{ 1M NaOH ad 10ml})$$

For HPAEC-analysis 50µl of a 1:400 dilution of the neutralized sample were injected (SC-HPAEC).

The following table shows the relative peak areas of alternan-oligosaccharides.

The results show that no degradation of oligosaccharides (DP 3-7) is detectable up to a temperature of 120°C in water and acetic acid (pH 3). In HCl (pH 1.5) no degradation is detectable up to a temperature of 95°C. No increase of glucose was detected (data not shown).

At a temperature of 120°C in HCl degradation was detected, shown by increase of detected glucose (data not shown) and shown by the decrease of relative peak area of DP4 - DP7 (see table).

However, it should be taken into account that the 120°C sample was heated for one hour due to technical reasons (autoclave), which is much longer than for the other samples (10 min).

|  | rel. area (%) | | | | | | |
|---|---|---|---|---|---|---|---|
| Sample Name | Panose | DP4-1 | DP4-2 | DP5 | DP6-1 | DP6-2 | DP7 |
| M2 H2O 20°C | 0.257 | 0.066 | 0.313 | 0.270 | 0.023 | 0.052 | 0.020 |
| M2 H2O 40°C | 0.257 | 0.066 | 0.313 | 0.269 | 0.023 | 0.052 | 0.019 |
| M2 H2O 60°C | 0.257 | 0.067 | 0.313 | 0.269 | 0.023 | 0.052 | 0.019 |
| M2 H2O 80°C | 0.260 | 0.057 | 0.317 | 0.271 | 0.023 | 0.052 | 0.019 |
| M2 H2O 95°C | 0.257 | 0.067 | 0.314 | 0.269 | 0.023 | 0.052 | 0.019 |
| M2 H2O 120°C | 0.255 | 0.075 | 0.311 | 0.267 | 0.022 | 0.051 | 0.019 |
|  |  |  |  |  |  |  |  |
| M2 HAc 20°C | 0.257 | 0.066 | 0.313 | 0.270 | 0.023 | 0.052 | 0.019 |
| M2 HAc 40°C | 0.259 | 0.057 | 0.316 | 0.272 | 0.023 | 0.052 | 0.020 |
| M2 HAc 60°C | 0.257 | 0.066 | 0.314 | 0.269 | 0.023 | 0.052 | 0.019 |
| M2 HAc 80°C | 0.257 | 0.066 | 0.314 | 0.269 | 0.023 | 0.051 | 0.019 |
| M2 HAc 95°C | 0.257 | 0.066 | 0.313 | 0.269 | 0.023 | 0.052 | 0.019 |
| M2 HAc 120°C | 0.260 | 0.065 | 0.314 | 0.268 | 0.022 | 0.051 | 0.019 |
|  |  |  |  |  |  |  |  |

(continued)

| Sample Name | rel. area (%) | | | | | | |
|---|---|---|---|---|---|---|---|
| | Panose | DP4-1 | DP4-2 | DP5 | DP6-1 | DP6-2 | DP7 |
| M2 HCl 20°C | 0.257 | 0.066 | 0.313 | 0.270 | 0.023 | 0.052 | 0.019 |
| M2 HCl 40°C | 0.256 | 0.066 | 0.314 | 0.269 | 0.023 | 0.052 | 0.019 |
| M2 HCl 60°C | 0.257 | 0.066 | 0.314 | 0.269 | 0.023 | 0.051 | 0.019 |
| M2 HCl 80°C | 0.260 | 0.057 | 0.317 | 0.271 | 0.023 | 0.052 | 0.020 |
| M2 HCl 95°C | 0.260 | 0.066 | 0.312 | 0.268 | 0.023 | 0.052 | 0.019 |
| *M2 HCl 120°C* | *0.397* | *0.055* | *0.258* | *0.229* | *0.018* | *0.031* | *0.012* |

**Claims**

1. Use of an alternan-oligosaccharide as a degradation-resistant ingredient for acidic beverages.

2. Use according to claim 1 wherein the beverage has a pH of 6 or below.

3. Use according to claim 1 wherein the beverage has a pH of 3.5 or below.

4. Use according to one of the preceding claims wherein the beverage is a clear beverage.

5. Use according to one of the preceding claims wherein the beverage is selected from fruit juices, energy drinks, lemonades, sherbets, sodas, soft drinks, and flavored waters.

6. Use according to one of the preceding claims wherein the alternan-oligosaccharide is produced by the reaction of sucrose with an acceptor molecule in presence of alternansucrase enzyme.

7. Use according to claim 6 wherein the acceptor molecule is selected from maltose, isomaltose, maltitol, (iso)maltotriose and methyl-$\alpha$-D-glucan.

8. Beverage comprising alternan-oligosaccharide as degradation-resistant ingredient.

9. Beverage according to claim 8 having a pH of 6 or below.

10. Beverage according to claim 8 having a pH of 3.5 or below.

11. Beverage according to one of the preceding claims which is a clear beverage.

12. Beverage according to one of the preceding claims which is selected from fruit juices, energy drinks, lemonades, sherbets, sodas, soft drinks, and flavored waters.

13. Beverage according to one of the preceding claims wherein the alternan-oligosaccharide is produced by the reaction of sucrose with an acceptor molecule in presence of alternansucrase enzyme.

14. Beverage according to claim 13 wherein the acceptor molecule is selected from maltose, isomaltose, maltitol, isomaltotriose and methyl-$\alpha$-D-glucan.

15. Beverage according to one of the preceding claims wherein the alternan-oligosaccharide is added in an amount of 1-20 weight-% based on the total weight.

Europäisches Patentamt

European Patent Office

Office européen des brevets

**EUROPEAN SEARCH REPORT**

Application Number

EP 08 10 1169

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEATHERS TIMOTHY D ET AL: "Characterization of a novel modified alternan." CARBOHYDRATE POLYMERS, vol. 54, no. 1, 1 October 2003 (2003-10-01), pages 107-113, XP004447713 ISSN: 0144-8617 * page 107, paragraphs 1,3 * ----- | 1-15 | INV. A23L1/30 A23L1/308 A23L1/09 A23L2/52 A23L1/054 C12N9/10 C12N9/24 C12P19/04 C12P19/18 |
| X | BEN HARDIN: "Nonfattening Food Additives - From Sugar?" AGRICULTURAL RESEARCH, SCIENCE AND EDUCATION ADMINISTRATION, WASHINGTON, DC, US, [Online] vol. 47, no. 9, 1 January 1999 (1999-01-01), pages 10-11, XP002486611 ISSN: 0002-161X Retrieved from the Internet: URL:http://genes.pp.ksu.edu/is/AR/archive/sep99/sugar0999.pdf> [retrieved on 2008-07-02] * page 10, last line , paragraph 1 - page 11, line R, paragraph 1 * ----- | 1-15 | |
| | | | TECHNICAL FIELDS SEARCHED (IPC) |
| X | WO 2007/011222 A (TNO [NL]; VAN GEEL-SCHUTTEN GERRITDINA H [NL]; EMEIS JOSEPUS JAN [NL]) 25 January 2007 (2007-01-25) * claim 1 * * page 4, paragraph 15 * * page 6, paragraphs 21,23 * ----- -/-- | 1-15 | A23L C12N C12P |

The present search report has been drawn up for all claims

| Place of search | Date of completion of the search | Examiner |
|---|---|---|
| The Hague | 2 October 2008 | Tallgren, Antti |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 08 10 1169

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | WO 2006/088884 A (CARGILL INC [US]; CARLSON TING L [US]; WOO ANTON [US]; ZHENG GUO-HUA []) 24 August 2006 (2006-08-24) <br> * claims 1,2,13,14,21,22; examples 4-8 * <br> * page 1, line 1 - page 2, line 21 * <br> ----- | 1-15 | |
| X | WO 03/008618 A (TNO [NL]; VAN GEEL-SCHUTTEN GERRITDINA H [NL]) 30 January 2003 (2003-01-30) <br> * page 1, line 4 - page 2, line 5 * <br> * page 5, paragraph 15 * <br> * page 7, paragraph 25; examples 4,5 * <br> ----- | 1-15 | |
| A | WO 2004/023891 A (CERESTAR HOLDING BV [NL]; VERCAUTEREN RONNY LEONTINA MAR [BE]; NGUYEN) 25 March 2004 (2004-03-25) <br> * claims 1,7-10; example 4 * <br> ----- | 1-15 | |
| A | WO 03/010177 A (NASA [US]) 6 February 2003 (2003-02-06) <br> * claims 6,14-17; figures 9,10 * <br> * page 3, paragraph 3-5 * <br> * page 9, paragraph 1 * <br> ----- | 1-15 | TECHNICAL FIELDS SEARCHED (IPC) |
| A | DE 102 09 629 B3 (STERN ENZYM GMBH & CO KG [DE]) 8 January 2004 (2004-01-08) <br> * claims 1,8; examples 1,2 * <br> ----- | 1-15 | |
| A | WO 2006/062410 A (TNO [NL]; VAN GEEL-SCHUTTEN GERRITDINA H [NL]; HEDDES CORNELIUS EGIDIU) 15 June 2006 (2006-06-15) <br> * page 4, paragraph 15; claim 1 * <br> * page 7, paragraph 24 * <br> ----- | 1-15 | |
| A | US 2006/141127 A1 (STEPHEN JEANETTE [US] ET AL) 29 June 2006 (2006-06-29) <br> * claim 1; examples 1,2 * <br> ----- | 1-15 | |

-/--

The present search report has been drawn up for all claims

| Place of search | Date of completion of the search | Examiner |
|---|---|---|
| The Hague | 2 October 2008 | Tallgren, Antti |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 08 10 1169

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2007/128559 A (BAYER CROPSCIENCE AG [DE]; MEUSER FRIEDRICH [DE]; BAUER INGO [DE]; HEL) 15 November 2007 (2007-11-15) * claims 1,18,23,31; examples 1,2 * ----- | 1-15 | |

**TECHNICAL FIELDS SEARCHED (IPC)**

The present search report has been drawn up for all claims

| Place of search | Date of completion of the search | Examiner |
|---|---|---|
| The Hague | 2 October 2008 | Tallgren, Antti |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 08 10 1169

This annex lists the patent family members relating to the patent documents cited in the above-mentioned European search report.
The members are as contained in the European Patent Office EDP file on
The European Patent Office is in no way liable for these particulars which are merely given for the purpose of information.

02-10-2008

| Patent document cited in search report | | Publication date | Patent family member(s) | | Publication date |
|---|---|---|---|---|---|
| WO 2007011222 | A | 25-01-2007 | NONE | | |
| WO 2006088884 | A | 24-08-2006 | AU | 2006214433 A1 | 24-08-2006 |
| | | | CA | 2597886 A1 | 24-08-2006 |
| | | | EP | 1856270 A1 | 21-11-2007 |
| | | | JP | 2008529534 T | 07-08-2008 |
| WO 03008618 | A | 30-01-2003 | CA | 2454563 A1 | 30-01-2003 |
| | | | JP | 2005500839 T | 13-01-2005 |
| | | | NZ | 530638 A | 27-01-2006 |
| | | | US | 2005059633 A1 | 17-03-2005 |
| WO 2004023891 | A | 25-03-2004 | AU | 2003267060 A1 | 30-04-2004 |
| WO 03010177 | A | 06-02-2003 | US | 2003022319 A1 | 30-01-2003 |
| | | | US | 6479275 B1 | 12-11-2002 |
| DE 10209629 | B3 | 08-01-2004 | NONE | | |
| WO 2006062410 | A | 15-06-2006 | NONE | | |
| US 2006141127 | A1 | 29-06-2006 | CA | 2529330 A1 | 23-06-2006 |
| WO 2007128559 | A | 15-11-2007 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7182954 B
- WO 2006088884 A
- WO 0047727 A
- US 5702942 A, Leathers

### Non-patent literature cited in the description

- **Cote ; Robyt.** *Carbohydrate Research,* 1982, vol. 111, 127-142
- **Arguello-Morales MA ; Remaud-Simeon M ; Pizzut S ; Sarcabal P ; Willemot R ; Monsan P.** Sequence analysis of the gene encoding alternansucrase, a sucrose glucosyltransferase from Leuconostoc mesenteroides NRRL B-1355. *FEMS Microbiol 1 Lett,* 2000, vol. 182, 81-85
- **Degenkolb J ; Takahashi M ; Ellestad GA ; Hillen W.** *Antimicrob. Agents Chemother,* 1991, vol. 35 (8), 1591-1595
- **Dotto GP ; Horiuchi K ; Zinder ND.** Initiation and termination of phage fl plus-strand synthesis. *Proc Natl Acad Sci U S A.,* 1982, vol. 79, 7122-7126
- **Horn, U. ; Strittmatter, W. ; Krebber, A. ; Knüpfer, U. ; Kujau, M. ; Wenderoth, R. ; Müller, K. ; Matzku, S. ; Plückthun, A. ; Riesenberg, D.** High volumetric yields of functional dimeric miniantibodies in Escherichia coli, using an optimized expression vector and high-cell-density fermentation under non-limited growth conditins. *Appl. Microbio. Biotechnol.,* 1996, vol. 46, 524-532
- **Jeong SH ; Bae IK ; Lee JH ; Sohn SG ; Kang GH ; Jeon GJ ; Kim YH ; Jeong BC ; Lee SH.** Molecular Characterization of Extended-Spectrum Beta-Lactamases Produced by Clinical Isolates of Klebsiella pneumoniae and Escherichia coli from a Korean Nationwide Survey. *J Clin Microbiol,* 2004, vol. 42, 2902-2906
- **López-Muguía, A. ; Pelenc, V. ; Remaud, M. ; Biton, J.1 ; Michel, J.M. ; Lang, C. ; Paul, F. ; Monsan, P.** Production and purification of alternansucrase, a glucosyltransferase from Leuconostoc mesenteroides NRRL B-1355, for the synthesis of oligoalternan. *Enzyme Microb. Technol.,* 1993, vol. 15, 77-85
- **Schmidt TGM ; Skerra A.** The random peptide library-assisted engineering of a C-terminal affinity peptide, useful for the detection and purification of a functional Ig Fv fragment. *Protein Eng,* 1993, vol. 6, 109-122
- **Skerra, A.** Use of the tetracycline promoter for the tightly regulated production of a murine antibody fragment in Escherichia coli. *Gene,* 1994, vol. 151, 131-135